# EUROPÄISCHE PATENTSCHRIFT

(11) **EP 3 077 357 B1**
(45) Veröffentlichungstag und Bekanntmachung des Hinweises auf die Patenterteilung: **08.08.2018**
(21) Anmeldenummer: 14799349.7
(22) Anmeldetag: 14.11.2014
(51) Int. Cl.: C07C 45/50, C07C 45/66, C07C 45/75, C07C 51/235, C07C 51/44, C07C 53/126, C07C 47/02, C07C 47/19, C07C 47/21

(54) **VERFAHREN ZUR HERSTELLUNG VON 2-METHYLBUTTERSÄURE MIT EINEM VERMINDERTEM GEHALT AN 3-METHYLBUTTERSÄURE AUS DEN BEI DER HERSTELLUNG VON PENTANSÄUREN ANFALLENDEN NEBENSTRÖMEN**
METHOD FOR PRODUCING 2-METHYLBUTYRIC ACID HAVING A REDUCED CONTENT OF 3-METHYLBUTYRIC ACID FROM THE SECONDARY FLOWS ARISING IN THE PRODUCTION OF PENTANOIC ACIDS
PROCÉDÉ DE PRÉPARATION D'ACIDE 2-MÉTHYLBUTYRIQUE PRÉSENTANT UNE TENEUR RÉDUITE EN ACIDE 3-MÉTHYLBUTYRIQUE À PARTIR DES FLUX SECONDAIRES PRODUITS LORS DE LA PRÉPARATION D'ACIDES PENTANOÏQUES

(30) Priorität: 05.12.2013 DE 102013020320
(43) Veröffentlichungstag der Anmeldung: 12.10.2016
(73) Patentinhaber: OXEA GmbH, 46147 Oberhausen (DE)
(72) Erfinder: THEUERKAUF, Jens, 47802 Krefeld (DE); STRUTZ, Heinz, 47445 Moers (DE)
(86) Internationale Anmeldenummer: PCT/EP2014/003056
(87) Internationale Veröffentlichungsnummer: WO 2015/082043

(56) Entgegenhaltungen:
- EP-A1- 1 854 778
- DE-A1- 3 744 212

## Beschreibung

Die vorliegende Erfindung betrifft ein Verfahren zur Herstellung von 2-Methylbuttersäure mit einem vermindertem Gehalt an 3-Methylbuttersäure aus Nebenströmen, die bei der Herstellung von Pentansäuren anfallen. Pentansäuren, auch Valeriansäuren genannt, haben als Zwischenprodukte in der industriellen organischen Chemie wirtschaftliche Bedeutung erlangt. Sie kommen in vier verschiedenen Strukturisomeren als lineare n-Pentansäure, verzweigte 2-Methylbuttersäure, verzweigte 3-Methylbuttersäure sowie als hochverzweigte Pivalinsäure vor. Pentansäuren können als solche verwendet werden, beispielsweise für die Herstellung von Riechstoffen. Daneben finden Ester der Pentansäuren mit Polyolen als Schmierstoffe zunehmende Bedeutung. Dabei können die Pentansäuren in reiner Isomerenform oder als Isomerengemisch verarbeitet werden (Weissermel, Arpe, Industrielle Organische Chemie, 3. Auflage, VCH Verlagsgesellschaft mbH, Weinheim, 1988, Seite 218; Schneidmeir, Chemiker-Zeitung, 96. Jahrgang (1972), Nr. 7, Seiten 383-387). Die isomeren Pentansäuren werden technisch durch Oxidation der entsprechenden Pentanale, oder im Falle der Pivalinsäure durch Hydrocarboxylierung, produziert. (Ullmann's Encyclopedia of Industrial Chemistry, 6th. Edition, 2003, Wiley-VCH Verlag GmbH & Co. KGaA, Weinheim, Vol. 6, Seiten 495-503).

Die Ausgangsverbindungen Pentanale werden technisch durch Umsetzung von Butenen mit Synthesegas, einem Gemisch aus Kohlenmonoxid und Wasserstoff, in Gegenwart von Übergangsmetallverbindungen hergestellt. Man bezeichnet die Reaktion von Olefinen mit Synthesegas auch als Hydroformylierungsreaktion oder Oxo-Reaktion und man erhält bei der Hydroformylierung von Buten-1 neben dem geradkettigen n-Aldehyd n-Pentanal auch gewisse Anteile an dem iso-Aldehyd 2-Methylbutanal (Ullmann's Encyclopedia of Industrial Chemistry, 6th. Edition, 2003, Wiley-VCH Verlag GmbH & Co. KGaA, Weinheim, Vol. 2, Seiten 73-74; Vol. 25, Seiten 286-289).

Butene werden technisch durch die Dampfspaltung von Naphtha gewonnen. Üblicherweise wird aus dem Butenschnitt der Naphthaspaltung zunächst 1,3-Butadien unter Bildung von Raffinat I und anschließend Isobuten unter Bildung von Raffinat II abgetrennt (Weissermel, Arpe, Industrielle Organische Chemie, 3. Auflage, VCH Verlagsgesellschaft mbH, 1988, Seiten 71-79). Für die nachfolgende Hydroformylierungsreaktion setzt man überwiegend Raffinat II ein, in dem ein geringer Isobutenrestanteil zugelassen werden kann. In Sonderfällen kann auch Raffinat I mit einem hohen Isobutenanteil verarbeitet werden, gelegentlich auch ein an Buten-1 abgereichertes Raffinat II, das man auch als Raffinat III bezeichnet.

Die Hydroformylierungsreaktion kann sowohl in Anwesenheit als auch in Abwesenheit von komplexbildenden Verbindungen, beispielsweise in Gegenwart von Organophosphorverbindungen, durchgeführt werden. Nach EP 0 366 089 A2 arbeitet man in einer homogenen organischen Lösung unter Katalyse von Rhodium-Triphenylphosphin. Da im Allgemeinen ein möglichst hoher Anteil an n-Pentanal gegenüber 2-Methybutanal im gebildeten Pentanalgemisch angestrebt wird, wird die Hydroformylierungsreaktion häufig in Gegenwart von homogen gelösten Übergangsmetallkomplexen durchgeführt, die zunächst eine Isomerisierung des Butens-2 in Buten-1 ermöglichen, das dann im überwiegenden Maße zu n-Pentanal hydroformyliert wird. Für die isomerisierende Hydroformylierung eines Gemisches linearer Butene geeignete Rhodiumkomplexkatalysatoren werden beispielsweise in DE 102 25 282 A1 beschrieben, in denen die Komplexliganden ein Xanthengerüst besitzen.

Rhodiumkomplexkatalysatoren auf Basis von Bisphosphit-Liganden zusammen mit sterisch gehinderten sekundären Aminen, die ebenfalls für die isomerisierende Hydroformylierung eines Gemisches linearer Butene geeignet sind, werden in DE 10 2008 002 187 A1 behandelt. Auch zweistufige Verfahrensvarianten sind bekannt, beispielsweise nach DE 43 33 324 A1, DE 42 10 026 A1, DE 101 08 474 A1 und DE 101 08 475. In der ersten Stufe wird vorzugsweise Buten-1 umgesetzt während in der zweiten Stufe das Buten-2-haltige Abgas aus der ersten Stufe zu einem Gemisch aus n-Pentanal und 2-Methylbutanal hydroformyliert wird. Nach DE 43 33 324 A1 und DE 101 08 474 A1 kann die erste Hydroformylierungsstufe auch in Gegenwart wasserlöslicher Rhodiumkomplexkatalysatoren durchgeführt werden. Bei dieser Art der Reaktionsführung liegt eine flüssige, wässrige Katalysatorlösung neben der flüssigen, organischen Reaktionslösung vor, die nach Verlassen der Hydroformylierungszone auf einfache Weise voneinander durch Phasentrennung separiert werden können. Aufgrund des Vorliegens einer wässrigen und einer organischen flüssigen Phase bezeichnet man diese Art der Reaktionsführung auch als heterogenes Verfahren oder Zweiphasenverfahren.

Je nach der Zusammensetzung des Buteneinsatzgemisches und den Reaktionsbedingungen in der Hydroformylierungsstufe erhält man ein Pentanalgemisch mit wechselnden Anteilen an n-Pentanal, 2-Methylbutanal, 3-Methylbutanal und geringen Menge an Pivalaldehyd, das üblicherweise aufdestilliert wird. Aufgrund der geringen Siedepunktsdifferenz zwischen 2-Methylbutanal (92°C bei Normaldruck) und 3-Methylbutanal (92,5°C bei Normaldruck) lässt sich 3-Methylbutanal bei vertretbarem Destillationsaufwand nicht vollständig vom 2-Methylbutanal abtrennen und das erhaltene 2-Methylbutanal wird mit einem Restgehalt an 3-Methylbutanal von üblicherweise größer als 0,2 Gew.-%, bezogen auf den organischer Anteil, für die Herstellung von 2-Methylbuttersäure verwendet. Für besondere Anwendungen, beispielsweise für die Riechstoffherstellung oder für spezielle Schmierstoffe, wird jedoch eine 2-Methylbuttersäurequalität nachgefragt, bei der der Restgehalt an 3-Methylbuttersäure unter 0,2 Gew.-%, bezogen auf den organischen Anteil, zu liegen hat. Ein sehr niedriger Restgehalt an 3-Methylbuttersäure unter 0,2 Gew.-% ist gegebenenfalls auch für n-Pentansäure/2-Methylbuttersäure-Mischungen wichtig. Es besteht daher Bedarf an einem Verfahren zur Herstellung von 2-Methylbuttersäure mit einem vermindertem Restgehalt an 3-Methylbuttersäure aus den bei der Pentansäureherstellung anfallenden Nebenströmen.

Die vorliegende Erfindung besteht daher in einem Verfahren zur Herstellung von 2-Methylbuttersäure mit einem vermindertem Gehalt an 3-Methylbuttersäure aus den bei der Herstellung von Pentansäuren anfallenden Nebenströmen. Es ist dadurch gekennzeichnet, dass man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Gemisch in einen mit 2-Methylbutanal und 3-Methylbutanal angereicherten Stoffstrom und in einen mit n-Pentanal angereicherten Stoffstrom trennt;
c) den gemäß Schritt b) mit 2-Methylbutanal und 3-Methylbutanal angereicherten Stoffstrom mit Formaldehyd umsetzt;
d) das nach Schritt c) erhaltene Umsetzungsprodukt mit einem Oxidationsmittel behandelt; und
e) aus dem gemäß Schritt d) erhaltenen Umsetzungsprodukt 2-Methylbuttersäure mit einem Restgehalt an 3-Methylbuttersäure von weniger als 0,2 Gew.-%, bezogen auf den organischen Anteil, destillativ entfernt,
mit der Maßgabe, dass man nach der Umsetzung mit Formaldehyd gemäß Schritt c) die organische Phase abtrennt und in Gegenwart eines Hydrierkatalysators mit Wasserstoff bei erhöhter Temperatur und erhöhtem Druck selektiv hydriert und den erhaltenen Hydrieraustrag nach Abtrennung des Hydrierkatalysators mit einem Oxidationsmittel gemäß Schritt d) behandelt.

Einsatzstoffe für das erfindungsgemäße Verfahren sind Kohlenwasserstoffgemische, die typischerweise keine oder sehr geringe Mengen an mehrfach ungesättigten Verbindungen und Acetylenverbindungen enthalten und mindestens eines der Olefine cis-Buten-2, trans-Buten-2 und Buten-1 enthalten. Darüber hinaus können in dem Einsatzgemisch wechselnde Anteile an Isobuten zugegen sein. Solche Einsatzgemische stehen als Raffinat I, Raffinat II oder Raffinat III technisch zur Verfügung.

Die Butenhydroformylierung kann dabei in homogener Variante in dem organischen Reaktionsmedium mit gelösten Übergangsmetallkatalysatoren der Gruppe VIII des Periodensystems der Elemente nach der unmodifizierten oder der mit Komplexliganden modifizierten Variante durchgeführt werden. In dem organische Reaktionsmedium haben sich als besonders wirksame Lösungsmittel die höher siedenden Kondensationsverbindungen der Pentanale, insbesondere die Trimeren, erwiesen, die als Nebenprodukte bei der Hydroformylierung anfallen, sowie ihre Mischungen mit den herzustellenden Pentanalen, so dass ein weiterer Lösungsmittelzusatz nicht unbedingt erforderlich ist. In einigen Fällen kann sich jedoch ein Lösungsmittelzusatz als zweckmäßig erweisen. Als Lösungsmittel werden organische Verbindungen eingesetzt, in denen Ausgangsmaterial, Reaktionsprodukt und Katalysator löslich sind. Beispiele für solche Verbindungen sind aromatische Kohlenwasserstoffe, wie Benzol und Toluol oder die isomeren Xylole und Mesitylen. Andere gebräuchliche Lösungsmittel sind Paraffinöl, Cyclohexan, n-Hexan, n-Heptan oder n-Octan, Ether wie Tetrahydrofuran, Ketone oder Texanol® der Firma Eastman. Wird bei der homogenen Variante in Gegenwart von Komplexliganden gearbeitet, eignen sich Triarylphosphine, wie Triphenylphosphin (EP 0 366 089 A2), Diphosphine, beispielsweise solche auf Basis des Xanthengerüstes (DE 102 25 282 A1), Phosphite, wie beispielsweise in US 4,599,206 beschrieben, oder Diphosphite, beispielsweise wie in EP 0 213 639 A2 und DE 10 2008 002 187 A1 beschrieben. Auch Mischungen von Komplexliganden, beispielsweise aus Triarylphopshinen mit Phosphiten oder Diphosphiten, wie aus WO 2010/117391 A1 bekannt, können in der Hydroformylierungsreaktion eingesetzt werden.

Durch die Wahl der Zusammensetzung des Buteneinsatzgemisches und der Hydroformylierungsbedingungen kann das Verhältnis von n-Pentanal zu 2-Methylbutanal gesteuert werden.

Wird ein möglichst hoher Anteil an n-Pentanal gegenüber 2-Methylbutanal im Hydroformylierungsgemisch angestrebt, empfiehlt sich neben einem Buten-1 reichen Einsatzstrom die Verwendung von modifizierten Übergangsmetallkatalysatoren, die zunächst eine Isomerisierung des Restgehalts an Buten-2 in Buten-1 bewirken, das dann im überwiegenden Maße zu n-Pentanal hydroformyliert wird. In einer Ausgestaltung des erfindungsgemäßen Verfahrens können die aus DE 102 25 282 A1 bekannten Hydroformylierungskatalysatoren mit Komplexliganden auf Basis des Xanthengerüstes oder die aus EP 0 213 639 A2 oder DE 10 2008 002 187 A1 bekannten Hydroformylierungskatalysatoren auf Basis sterisch gehinderter Diphosphite eingesetzt werden.

Hohe Anteile an n-Pentanal können auch in einer zweistufigen Verfahrensvariante erhalten werden, die aus DE 101 08 474 A1 und DE 101 08 475 an sich bekannt ist. In der ersten Stufe, die ebenfalls nach der heterogenen Variante in Gegenwart von Wasser mit wasserlöslichen Komplexliganden, beispielsweise mit sulfonierten Phosphinen wie Triphenylphosphin mit unterschiedlichem Sulfonierungsgrad, durchgeführt werden kann, reagiert überwiegend Buten-1 in hoher Selektivität zu n-Pentanal und das mit Buten-2 angereicherte Abgas wird anschließend in einer zweiten Stufe unter isomersierenden Bedingungen zu einem Pentanalgemisch mit einem hohen n-Pentanalanteil umgesetzt. Durch Vereinigung der Stoffströme aus der ersten und zweiten Hydroformylierungsstufe kann ein Pentanalgemisch mit einem hohen Anteil n-Pentanal zu 2-Methylbutanal hergestellt werden.

Wird ein hoher Anteil an 2-Methylbutanal im Pentanalgemisch angestrebt, beispielsweise aufgrund von Marktgegebenheiten, oder er ergibt sich aufgrund der Zusammensetzung des Buteneinsatzgemisches, arbeitet man bevorzugt in Abwesenheit von Komplexliganden nach der unmodifizierten Fahrweise. Dabei bildet sich der aktive Hydroformylierungskatalysator aus dem Übergangsmetall oder der Übergangsmetallverbindung und Kohlenmonoxid. Es wird in der Fachliteratur angenommen, dass die Übergangsmetallverbindung HM(CO)₄ die katalytisch aktive Übergangsmetallspezies bei der unmodifizierten Übergangsmetallkatalyse ist.

Mit zunehmendem Isobutengehalt in dem Buteneinsatzgemisch nimmt auch der Anteil an 3-Methylbutanal in dem Hydroformylierungsprodukt zu.

Bei der modifizierten Variante beträgt das molare Verhältnis von Übergangsmetall zu Komplexligand im Allgemeinen 1:1 bis 1:1000, es kann aber auch noch höher liegen. Bevorzugt setzt man das Übergangsmetall und den Komplexliganden in einem molaren Verhältnis von 1:3 bis 1:500, vorzugsweise 1:50 bis 1:300 ein. Die modifizierte Hydroformylierungsreaktion des Butengemisches wird üblicherweise bei Temperaturen von 50 bis 160°C und Drücken von 0,2 bis 15 MPa durchgeführt. Die Übergangsmetallkonzentration beträgt im Allgemeinen 10 bis 700 ppm, vorzugsweise 25 bis 500 ppm, bezogen auf die Reaktionsmischung.

Wird nach der unmodifizierten Variante gearbeitet, setzt man das Übergangsmetall in geringeren Mengen ein, im Allgemeinen in einer Menge von 1 bis 100 ppm, vorzugsweise 2 bis 30 ppm, bezogen auf die eingesetzte Butenmenge. Man arbeitet zweckmäßigerweise bei höheren Drücken im Bereich von 5 bis 70 MPa, vorzugsweise von 5 bis 60 MPa und insbesondere von 10 bis 30 MPa. Geeignete Reaktionstemperaturen bewegen sich im Bereich von 50 bis 180°C, bevorzugt von 50 bis 150°C und insbesondere von 100 bis 150°C.

Die Zusammensetzung des Synthesegases kann in weiten Grenzen variiert werden. Im Allgemeinen setzt man Gemische ein, in denen das Molverhältnis von Kohlenmonoxid zu Wasserstoff 5:1 bis 1:5 beträgt. Üblicherweise ist dieses Verhältnis 1:1 oder weicht von diesem Wert zugunsten von Wasserstoff nur wenig ab. Das Gemisch enthaltend lineare Butene kann als solches oder in Lösung mit organischen Lösungsmitteln, wie Kohlenwasserstoffen, der Reaktionszone zugeführt werden.

Vorzugsweise verwendet man als Übergangsmetalle der Gruppe VIII des Periodensystems der Elemente Kobalt, Rhodium, Iridium, Nickel, Palladium, Platin, Eisen oder Ruthenium und insbesondere Rhodium und Kobalt. Der modifizierte oder nicht modifizierte Übergangsmetallkatalysator bildet sich unter den Bedingungen der Hydroformylierungsreaktion aus den eingesetzten Übergangsmetallverbindungen, wie deren Salzen, wie Chloriden, Nitraten, Sulfaten, Acetaten, Pentanoaten oder 2-Ethylhexanoaten, deren Chalkogeniden, wie Oxiden oder Sulfiden, deren Carbonylverbindungen, wie M₂(CO)₈, M₄(CO)₁₂, M₆(CO)₁₆, M₂(CO)₉, M₃(CO)₁₂, deren Organoübergangsmetallverbindungen, wie Carbonylacetylacetonaten oder Cyclooctadienylacetaten oder -chloriden. Dabei kann die Übergangsmetallverbindung als Feststoff oder zweckmäßigerweise in Lösung eingesetzt werden. Als Übergangsmetallverbindung, die als Katalysatorvorstufe verwendet wird, eignet sich insbesondere Rhodiumpentanoat, Rhodiumacetat, Rhodium-2-ethylhexanoat oder Kobaltpentanoat, Kobaltacetat oder Kobalt-2-ethylhexanoat oder Co₂(CO)₈, Co₄(CO)₁₂, Rh₂(CO)₈, Rh₄(CO)₁₂ oder Rh₆(CO)₁₆ oder Cyclopentadienylrhodiumverbindungen, Rhodiumacetylacetonat oder Rhodiumdicarbonylacetylacetonat. Bevorzugt werden Rhodiumoxid und insbesondere Rhodiumacetat, Rhodium-2-ethylhexanoat und Rhodiumpentanoat eingesetzt.

Es ist aber auch möglich, den Übergangsmetallkatalysator in einer Vorcarbonylierungsstufe zunächst zu präformieren und ihn anschließend der eigentlichen Hydroformylierungsstufe zuzuführen. Die Bedingungen der Präformierung entsprechen dabei im Allgemeinen den Hydroformylierungsbedingungen.

Die Hydroformylierungsstufe kann sowohl diskontinuierlich als auch kontinuierlich durchgeführt werden. Die Abtrennung des gebildeten Pentanalgemisches von dem Hydroformylierungskatalysator erfolgt nach konventionellen Verfahren, beispielsweise durch Destillation bei der homogenen Verfahrensführung oder durch einfache Phasentrennung von der wässrigen Katalysatorlösung bei der heterogenen oder zweiphasigen Verfahrensführung.

Der Übergangsmetallkatalysator wird gegebenenfalls nach Zusatz von frischer Übergangsmetallverbindung sowie gegebenenfalls von Frischligand, falls nach der modifizierten Fahrweise gearbeitet wird, und nach Entnahme eines Teils der im Verlauf der Reaktion gebildeten Aldehydkondensationsprodukte in die Reaktionszone zurückgeführt.

Die Forderung nach einer bestimmten Zusammensetzung des erhaltenen Pentanalgemisches bezüglich der Isomeren n-Pentanal, 2-Methylbutanal und 3-Methylbutanal richtet sich nach den Marktgegebenheiten und kann durch die Zusammensetzung des Einsatzbutengemisches sowie durch die Wahl der Hydroformylierungsbedingungen gesteuert werden. Häufig wird ein Pentanalgemisch angestrebt, das im Allgemeinen mindestens 85 mol-% n-Pentanal, weniger als 15 mol-% 2-Methylbutanal und in Abhängigkeit vom Isobutengehalt weniger als 5 mol-% 3-Methylbutanal, vorzugsweise weniger als 1 mol-% und insbesondere weniger als 0,2 mol-% 3-Methylbutanal enthält, jeweils bezogen auf die Summe an Pentanalen. Aber auch Pentanalgemische mit einem höheren Anteil an 2-Methylbutanal können vom Markt gefordert werden.

Das nach der Hydroformylierungsstufe und nach Katalysatorabtrennung erhaltene Pentanalgemisch, das man auch als rohes Hydroformylierungsprodukt ansehen kann, wird anschließend in einen mit 2-Methylbutanal und 3-Methylbutanal angereicherten leichter flüchtigen Stoffstrom und in einen mit n-Pentanal angereicherten schwerer flüchtigen Stoffstrom aufgetrennt, zweckmäßigerweise durch Destillation. Die Destillation des rohen Hydroformylierungsprodukts erfolgt nach konventionellen Verfahren an einer Destillationskolonne. Um möglichst reines n-Pentanal im schwerer flüchtigen Stoffstrom anzureichern, wird die Trennschärfe in der Destillation im Allgemeinen so gewählt, dass der erhaltene mit 2-Methylbutanal und mit 3-Methylbutanal angereicherte leichter flüchtige Stoffstrom ebenfalls noch Mengen an n-Pentanal enthält. Seine genaue Zusammensetzung hängt von der Zusammensetzung des Einsatzbutengemisches, den Hydroformylierungsbedingungen und den Destillationsbedingungen ab, beispielsweise kann die Zusammensetzung dieses flüchtigen Stoffstromes bei 80 bis 85 mol-% 2-Methylbutanal, 10 bis 14 mol-% n-Pentanal und 1 bis 10 mol-% 3-Methylbutanal, bezogen auf den Pentanalgehalt, liegen. Je nach Isobutengehalt im Einsatzbutengemisch kann der 3-Methylbutanalgehalt aber auch höher oder niedriger sein. Im schwerer flüchtigen Stoffstrom wird n-Pentanal nahezu frei von den übrigen Pentanalisomeren aufkonzentriert.

Während n-Pentanal mit einem Siedepunkt von 103°C bei Normaldruck aus diesem flüchtigen Stoffstrom durch eine weitere konventionelle Destillation an einer weiteren Kolonne mit 10 bis 100 Böden als Produkt hoher Reinheit abgetrennt werden kann, besitzen 2-Methylbutanal, das einen Siedepunkt von 92°C bei Normaldruck hat, und 3-Methylbutanal, das bei Normaldruck bei 92,5°C siedet, eine zu geringe Siedepunktsdifferenz für eine ausreichende Trennung bei vertretbarem Destillationsaufwand. Diese weitere Destillation zur n-Pentanalabtrennung kann optional durchgeführt werden.

Erfindungsgemäß wird der nach Destillation des rohen Hydroformylierungsprodukts erhaltene leichter flüchtige Stoffstrom mit dem Hauptbestandteil 2-Methylbutanal und den Restgehalten an 3-Methylbutanal und n-Pentanal mit Formaldehyd versetzt. Die Behandlung von α-alkylsubstituierten Aldehyden mit Formaldehyd zur Entfernung von Restmengen an Aldehyden mit zwei Wasserstoffatomen am α-ständigen Kohlenstoffatom relativ zur Carbonylgruppe im Zuge der Aufreinigung wird auch als Methylenierungsreaktion bezeichnet und ist aus dem Stand der Technik an sich bekannt und wird beispielsweise in DE 3842186 A1 und DE 3744212 A1 beschrieben.

Das 2-Methylbutanal, 3-Methylbutanal und n-Pentanal enthaltende Gemisch wird mit Formaldehyd umgesetzt in Gegenwart eines Aldolisierungskatalysators, typischerweise ein Gemisch aus einem sekundären Amin, beispielsweise einem Amin der allgemeinen Formel R¹-NH-R², wobei R¹ und R² gleich oder verschieden sind und für Akylreste mit 1 bis 12, vorzugsweise 3 bis 5 Kohlenstoffatomen stehen, und einer Monocarbonsäure mit 1 bis 10 Kohlenstoffatomen oder einer Di- oder Polycarbonsäure mit 2 bis 10 Kohlenstoffatomen. Vorzugsweise verwendet man als Aldolisierungskatalysator ein Gemisch aus Di-n-butylamin und n-Buttersäure. Andere Aldolisierungskatalysatoren für den Methylenierungsschritt sind jedoch nicht ausgeschlossen. Formaldehyd wird in fester Form, als Paraformaldehyd, oder zweckmäßigerweise als wässrige Lösung in handelsüblicher Konzentration, wie 30 bis 50 Gew.-%ig, verwendet, wobei das molare Verhältnis von Formaldehyd zu der Summe an Aldehyden mit zwei Wasserstoffatomen am α-ständigen Kohlenstoffatom relativ zur Carbonylgruppe 1 bis 2 beträgt.

Die Umsetzung wird üblicherweise bei Temperaturen von 0 bis 100°C bei Eigendruck oder leichtem Überdruck durchgeführt. Als Reaktoren eignen sich die in der chemischen Verfahrenstechnik üblichen Aggregate, wie Rührkessel, Rührkesselkaskaden, Mischpumpen oder Strömungsrohre. Sowohl eine absatzweise als auch kontinuierliche Reaktionsführung ist möglich. Besonders eignen sich für die kontinuierliche Reaktionsführung Strömungsrohre, beispielsweise ein senkrechts stehendes oder ein waagerecht angeordnetes Strömungsrohr oder ein mehrfach geschlägeltes Strömungsrohr. Das Strömungsrohr kann als Leerrohr betrieben werden, es kann jedoch ebenfalls Füllkörper oder Einbauten zur intensiven Durchmischung der organischen Phase mit der wässrigen Phase enthalten, beispielsweise Raschigringe, Sättel, Pallringe, Wendel, Strombrecher oder statische Mischer oder Mischerpackungen. Statische Mischelemente stehen kommerziell zur Verfügung und werden beispielsweise als Sulzermischer oder Kenicksmischer angeboten. Es hat sich eine Belastung des Strömungsrohrs mit dem Gemisch aus organischer Phase enthaltend 2-Methylbutanal, 3-Methylbutanal sowie n-Pentanal und wässriger Formaldehydlösung von 0,1 bis 10 h⁻¹, vorzugsweise von 0,1 bis 5 h⁻¹, und insbesondere von 0,1 bis 3h⁻¹ bezogen auf Reaktorvolumen und Zeit, als zweckmäßig erwiesen.

Das das Reaktionsgefäß verlassende Gemisch wird in einen Phasentrenner geleitet, in dem sich die organische Phase von der wässrigen Phase trennt.

Bei der Methylenierung bildet sich zunächst aus Formaldehyd und dem sekundären Amin im sauren Medium ein Mannich-Salz, das bevorzugt mit 3-Methylbutanal und n-Pentanal über die Bildung des α-Methylolderivats in Isopropylacrolein und n-Propylacrolein dehydratisiert, während 2-Methylbutanal unverändert bleibt. Isopropylacrolein besitzt einen Siedepunkt von 108,5°C bei Normaldruck und n-Propylacrolein siedet bei 117°C bei Normaldruck und beide Methylenierungsprodukte können von dem 2-Methylbutanal destillativ nach konventionellen Verfahren abgetrennt werden. Der 3-Methylbutanalgehalt im abgetrennten 2-Methylbutanal liegt dabei im Allgemeinen unter 0,2 Gew.-%, bezogen auf den organischen Anteil. Gegebenenfalls schließt sich nochmals eine Feindestillation des 2-Methylbutanals an.

Das erhaltene gereinigte 2-Methylbutanal wird anschließend zu 2-Methylbuttersäure oxidiert. Die Oxidation von 2-Methylbutanal ist an sich bekannt (Ullmann's Encyclopedia of Industrial Chemistry, 6th. Edition, 2003, Wiley-VCH Verlag GmbH & Co. KGaA, Weinheim, Vol. 6, Seiten 497-502) und wird vorzugsweise in der Flüssigphase durchgeführt, beispielsweise in Rohrrektoren, die mit einem Anströmboden versehen sind, obwohl andere Verfahrensausgestaltungen wie die Oxidation in der Gasphase nicht ausgeschlossen sind. Als Oxidationsmittel eignen sich übliche, zur Oxidation von aliphatischen Aldehyden geeignete Verbindungen wie Sauerstoff, sauerstoffenthaltende Gasgemische, Ozon, ozonhaltige Gasgemische, Peroxide, Persäuren, Metallsalze von Persäuren oder Übergangsmetalle in hohen Oxidationsstufen, beispielsweise Kaliumpermanganat oder Braunstein. Aufgrund der guten Verfügbarkeit verwendet man als Oxidationsmittel zweckmäßig molekularen Sauerstoff oder Gasgemische, die molekularen Sauerstoff enthalten. Weitere Bestandteile derartiger Gasgemische sind inerte Gase, z. B. Stickstoff, Edelgase und Kohlendioxid. Der Anteil der inerten Bestandteile des sauerstoffenthaltenden Gasgemisches beträgt bis zu 90 Vol-%, insbesondere 30 bis 80 Vol-%. Die bevorzugten Oxidationsmittel sind Sauerstoff oder Luft.

Die Oxidation kann entweder unter Katalysatorzusatz oder in Abwesenheit von Katalysatoren durchgeführt werden. Als Katalysatoren eignen sich Übergangsmetalle oder Verbindungen von Übergangsmetallen, die in geringen Mengen wie beispielsweise von 0,1 bis 5 ppm, berechnet als Übergangsmetall und bezogen auf eingesetzten Aldehyd, zugesetzt werden können wie Titan, Vanadium, Chrom, Molybdän, Mangan, Eisen, Kobalt, Nickel, Ruthenium, Rhodium, Palladium oder Kupfer. Eine solche Verfahrensführung wird beispielsweise in DE 100 10 771 C1 oder DE 26 04 545 A1 beschrieben.

Ebenfalls kann die Umsetzung in Anwesenheit von Alkali- oder Erdalkalimetallsalzen schwacher Säuren durchgeführt werden. Insbesondere bei der Oxidation von α-verzweigten Aldehyden, bei denen das dem Carbonylkohlenstoffatom benachbarte Kohlenstoffatom die Verzweigung trägt, empfiehlt der Stand der Technik die Anwesenheit von geringen Mengen an Alkalimetallcarboxylaten zur Selektivitätsverbesserung (DE 950 007, DE 100 10 771 C1). Zweckmäßigerweise wird die Oxidation in Gegenwart von 1 bis 30 mmol, vorzugsweise 1 bis 15 mmol und insbesondere 1 bis 8 mmol, je Mol Aldehyd, berechnet als Alkali- oder Erdalkalimetall, durchgeführt. Auch kann eine Kombination von Alkali- oder Erdalkalimetallcarboxylaten mit Übergangsmetallverbindungen, wie in EP 1 854 778 A1 behandelt, verwendet werden.

Es ist nicht erforderlich, die Alkali- oder Erdalkalimetallcarboxylate als einheitliche Verbindung einzusetzen. Es ist ebenfalls möglich, Gemische dieser Verbindungen zu verwenden, wobei man jedoch zweckmäßigerweise 2-Methylbutyrate verwendet. Vorzugsweise setzt man jedoch einheitliche Verbindungen ein, beispielsweise Lithium-, Kalium-, Natrium-, Calcium- oder Barium-2 -Methylbutyrat.

Im Allgemeinen stellt man eine Alkali- oder Erdalkalimetall-2-Methylbutyrate enthaltende Lösung durch Neutralisation einer wässrigen, die Alkali- oder Erdalkalimetallverbindung enthaltenden Lösung mit einem Überschuss an 2-Methylbuttersäure her und setzt diese Lösung dem zu oxidierenden 2-Methylbutanal zu. Als Alkali- oder Erdalkalimetallverbindungen eignen sich besonders die Hydroxide, Carbonate oder Hydrogencarbonate.

Es ist aber auch möglich, die Alkali- oder Erdalkalimetall-2-Methylbutyrate im Reaktionsgemisch zu erzeugen, indem man Alkali- oder Erdalkalimetallverbindungen zusetzt, die unter den Reaktionsbedingungen in die 2-Methylbutyrate überführt werden. Beispielsweise lassen sich Alkali- oder Erdalkalimetallhydroxide, -carbonate, -hydrogencarbonate oder -oxide in der Oxidationsreaktion einsetzen. Ihr Zusatz kann entweder in fester Form oder als wässrige Lösung erfolgen.

Die Umsetzung mit dem Oxidationsmittel, vorzugsweise mit Sauerstoff oder sauerstoffenthaltenden Gasen, wird in einem Temperaturbereich von 20 bis 100°C durchgeführt. Vorzugsweise arbeitet man zwischen 20 und 80°C, insbesondere zwischen 40 und 80°C. Die Temperaturführung, konstante oder variable Temperatur, kann den individuellen Erfordernissen des Ausgangs. materials und den Reaktionsgegebenheiten angepasst werden.

Die Umsetzung der Reaktionspartner erfolgt bevorzugt bei Atmosphärendruck. Die Anwendung erhöhten Drucks ist jedoch nicht ausgeschlossen. Üblicherweise arbeitet man in einem Bereich von Atmosphärendruck bis 1,5 MPa, vorzugsweise bei Atmosphärendruck bis 0,8 MPa.

Der Oxidationsschritt kann absatzweise oder kontinuierlich durchgeführt werden. Eine Rückführung nicht umgesetzter Reaktionsteilnehmer ist in beiden Fällen möglich.

Die erhaltene Säure wird anschließend destillativ zu spezifikationsgerechter Ware aufdestilliert. Die Reinheit der 2-Methylbuttersäure liegt im Allgemeinen bei über 99,7 Gew.-% und der Restgehalt an 3-Methylbuttersäure bei weniger als 0,2 Gew.-%, bezogen auf den organischen Anteil. 2-Methylbuttersäure dieser Qualität eignet sich in hervorragender Weise als Säurekomponente für die Herstellung von Esterschmierstoffen und Riechstoffen.

Nach dem erfindungsgemäßen Verfahren wird nach der Umsetzung des mit 2-Methylbutanal und 3-Methylbutanal angereicherten flüchtigen Stoffstroms, der ebenfalls noch Restmengen an n-Pentanal enthält, mit Formaldehyd in Gegenwart des Aldolisierungskatalysators gemäß Schritt c) die organische Phase von der wässrigen Phase abtrennt und die rohe organische Phase in Gegenwart eines Hydrierkatalysators mit Wasserstoff bei erhöhter Temperatur und erhöhtem Druck selektiv hydriert. Dabei wird die Doppelbindung in der α,β-Position relativ zum Carboxylkohlenstoffatom unter Beibehaltung der Aldehydgruppe abgesättigt, so dass als Ergebnis der Selektivhydrierung ein Gemisch enthaltend unverändertes 2-Methylbutanal, 2-Methylpentanal aus der Selektivhydrierung von n-Propylacrolein sowie 2,3-Dimethylbutanal aus der Selektivhydrierung von isoPropylacrolein erhalten wird. Die Selektivhydrierung wird in bekannter Weise an geträgerten oder ungeträgerten Katalysatoren, die als hydrieraktive Komponente Palladium, Platin, Rhodium und/oder Nickel enthalten, durchgeführt. Vorzugsweise arbeitet man mit Palladiumkatalysatoren bei Temperaturen von 120 bis 180°C, vorzugsweise 140 bis 160°C und bei einem Druck von 1,5 bis 5 MPa, vorzugsweise bei 2 bis 3 MPa.

Der erhaltene Hydrieraustrag wird vom Hydrierkatalysator gegebenenfalls abgetrennt und anschließend, gegebenenfalls nach Abtrennung von Leicht- und Schwersiedern, oxidiert. Die Oxidationsbedingungen entsprechend den zuvor genannten Bedingungen. Man erhält ein Rohsäuregemisch, das neben 2-Methylbuttersäure, Siedepunkt 177°C, noch 2-Methylpentansäure, Siedepunkt 196°C, und 2,3-Dimethylbuttersäure, Siedepunkt 191°C, jeweils bei Normaldruck, enthält. Nach Destillation unter konventionellen Bedingungen wird 2-Methylbuttersäure mit einer Reinheit von mehr als 99,7 Gew.-% und mit einem Restgehalt an 3-Methylbuttersäure von weniger als 0,2 Gew.-%, bezogen auf den organischen Anteil, erhalten.

In einer Ausgestaltung des erfindungsgemäßen Verfahrens wird nach Abtrennung des Hydrierkatalysators der Austrag aus der Selektivhydrierung fraktioniert destilliert. 2-Methylbutanal wird als leichter flüchtige Komponente vom 2-Methylpentanal und gegebenenfalls vorhandenen 2,3-Dimethylbutanal abgetrennt. Die getrennten Aldehydfraktionen wurden anschließend gemäß den zuvor beschriebenen Bedingungen mit einem Oxidationsmittel behandelt. Nach destillativer Reinigung erhält man 2-Methylbuttersäure mit einem Restgehalt an 3-Methylbuttersäure von weniger als 0,2 Gew.-%, bezogen auf den organischen Anteil. Als separate Fraktion erhaltenen 2-Methylpentansäure, die ebenfalls noch 2,3-Dimethylbuttersäure enthalten kann.

Das erfindungsgemäße Verfahren ermöglicht die Herstellung von 2-Methylbuttersäure in hoher Qualität aus Nebenströmen, die bei der Herstellung von Pentanalen anfallen. Aufgrund des geringen Restgehalts an 3-Methylbuttersäure eignet sich die erhaltene 2-Methylbuttersäure hervorragend als Säurekomponente in Esterverbindungen. Esterverbindungen mit niedrigen Monoalkoholen werden für die Herstellung von Riechstoffen eingesetzt während Esterverbindungen aus Polyolen für die Herstellung von synthetischen Schmierstoffen verwendet werden.

Die hochreine 2-Methylbuttersäure mit einem 3-Methylbuttersäuregehalt kleiner 0,2 Gew.-% kann ebenso mit hochreiner n-Pentansäure zu einem hochreinen Säuregemisch, das man auch als iso-Pentansäure bezeichnen kann, verarbeitet werden.

## Patentansprüche

1. Verfahren zur Herstellung von 2-Methylbuttersäure mit einem vermindertem Gehalt an 3-Methylbuttersäure aus den bei der Herstellung von Pentansäuren anfallenden Nebenströmen, **dadurch gekennzeichnet, dass** man
a) ein Gemisch enthaltend lineare Butene in Gegenwart von Übergangsmetallverbindungen der Gruppe VIII des Periodensystems der Elemente mit Kohlenmonoxid und Wasserstoff bei erhöhter Temperatur und erhöhtem Druck zu einem Pentanalgemisch umsetzt;
b) das gemäß Schritt a) erhaltene Gemisch in einen mit 2-Methylbutanal und 3-Methylbutanal angereicherten Stoffstrom und in einen mit n-Pentanal angereicherten Stoffstrom trennt;
c) den gemäß Schritt b) mit 2-Methylbutanal und 3-Methylbutanal angereicherten Stoffstrom mit Formaldehyd umsetzt;
d) das nach Schritt c) erhaltene Umsetzungsprodukt mit einem Oxidationsmittel behandelt; und
e) aus dem gemäß Schritt d) erhaltenen Umsetzungsprodukt 2-Methylbuttersäure mit einem Restgehalt an 3-Methylbuttersäure von weniger als 0,2 Gew.-%, bezogen auf den organischen Anteil, destillativ entfernt;
mit der Maßgabe, dass man nach der Umsetzung mit Formaldehyd gemäß Schritt c) die organische Phase abtrennt und in Gegenwart eines Hydrierkatalysators mit Wasserstoff bei erhöhter Temperatur und erhöhtem Druck selektiv hydriert und den erhaltenen Hydrieraustrag nach Abtrennung des Hydrierkatalysators mit einem Oxidationsmittel gemäß Schritt d) behandelt.

2. Verfahren gemäß Anspruch 1, **dadurch gekennzeichnet, dass** die Umsetzung mit Formaldehyd gemäß Schritt c) in Gegenwart eines sekundären Amins und einer Mono, Di- oder Polycarbonsäure erfolgt.

3. Verfahren gemäß Anspruch 1, **dadurch gekennzeichnet, dass** man den erhaltenen Hydrieraustrag nach Abtrennung des Hydrierkatalysators fraktioniert destilliert und die getrennten Fraktionen mit einem Oxidationsmittel gemäß Schritt d) behandelt.

4. Verfahren gemäß einem oder mehreren der Ansprüche 1 bis 3, **dadurch gekennzeichnet, dass** man als sekundäres Amin ein Alkylamin der Formel R¹-NH-R² verwendet, wobei R¹ und R² gleich oder verschieden sind und für Akylreste mit 1 bis 12, vorzugsweise 3 bis 5 Kohlenstoffatomen stehen.

5. Verfahren gemäß einem oder mehreren der Ansprüche 1 bis 4, **dadurch gekennzeichnet, dass** man eine Monocarbonsäure mit 1 bis 10 Kohlenstoffatomen oder eine Di- oder Polycarbonsäure mit 2 bis 10 Kohlenstoffatomen verwendet.

6. Verfahren gemäß einem oder mehreren der Ansprüche 1 bis 5, **dadurch gekennzeichnet, dass** die Oxidation in Schritt d) in Anwesenheit von Alkali- oder Erdalkalimetallcarboxylaten erfolgt.

7. Verfahren gemäß Anspruch 6, **dadurch gekennzeichnet, dass** man als Alkali- oder Erdalkalimetallcarboxylate Lithium-, Kalium-, Natrium-, Calcium- oder Bariumcarboxylate verwendet.

8. Verfahren gemäß einem oder mehreren der Ansprüche 1 bis 7, **dadurch gekennzeichnet, dass** man in Schritt d) in der Flüssigphase oxidiert.

9. Verfahren gemäß einem oder mehreren der Ansprüche 1 bis 8, **dadurch gekennzeichnet, dass** man in Schritt d) als Oxidationsmittel Sauerstoff oder sauerstoffenthaltende Gase verwendet.

10. Verfahren gemäß einem oder mehreren der Ansprüche 1 bis 9, **dadurch gekennzeichnet, dass** man in Schritt c) die Umsetzung mit Formaldehyd kontinuierlich in einem Strömungsrohr durchführt.

## Claims

1. Process for preparing 2-methylbutyric acid having a reduced content of 3-methylbutyric acid from the secondary streams obtained in the preparation of pentanoic acids, **characterized in that**
a) a mixture comprising linear butenes is reacted in the presence of transition metal compounds of group VIII of the Periodic Table of the Elements with carbon monoxide and hydrogen at elevated temperature and elevated pressure to give a pentanal mixture;
b) the mixture obtained in step a) is separated into a stream enriched with 2-methylbutanal and 3-methyl-butanal, and a stream enriched with n-pentanal;
c) the stream enriched with 2-methylbutanal and 3-methylbutanal of step b) is reacted with formaldehyde;
d) the reaction product obtained after step c) is treated with an oxidizing agent; and
e) 2-methylbutyric acid having a residual 3-methylbutyric acid content of less than 0.2% by weight, based on the organic component, is removed by distillation from the reaction product obtained in step d);
with the proviso that the reaction with formaldehyde in step c) is followed by removal of the organic phase and selective hydrogenation in the presence of a hydrogenation catalyst with hydrogen at elevated temperature and elevated pressure and, after removal of the hydrogenation catalyst, treatment of the hydrogenation output obtained with an oxidizing agent in step d).

2. Process according to Claim 1, **characterized in that** the reaction with formaldehyde in step c) is effected in the presence of a secondary amine and a mono-, di- or polycarboxylic acid.

3. Process according to Claim 1, **characterized in that**, after removal of the hydrogenation catalyst, the hydrogenation output obtained is fractionally distilled and the separated fractions are treated with an oxidizing agent in step d).

4. Process according to one or more of Claims 1 to 3, **characterized in that** the secondary amine used is an alkylamine of the formula R¹-NH-R² where R¹ and R² are the same or different and are each alkyl radicals having 1 to 12 and preferably 3 to 5 carbon atoms.

5. Process according to one or more of Claims 1 to 4, **characterized in that** a monocarboxylic acid having 1 to 10 carbon atoms or a di- or polycarboxylic acid having 2 to 10 carbon atoms is used.

6. Process according to one or more of Claims 1 to 5, **characterized in that** the oxidation in step d) is effected in the presence of alkali metal or alkaline earth metal carboxylates.

7. Process according to Claim 6, **characterized in that** the alkali metal or alkaline earth metal carboxylates used are lithium carboxylate, potassium carboxylate, sodium carboxylate, calcium carboxylate or barium carboxylate.

8. Process according to one or more of Claims 1 to 7, **characterized in that** oxidation is effected in the liquid phase in step d).

9. Process according to one or more of Claims 1 to 8, **characterized in that** the oxidizing agent used in step d) is oxygen or oxygen-containing gases.

10. Process according to one or more of Claims 1 to 9, **characterized in that** the reaction with formaldehyde in step c) is conducted continuously in a flow tube.

## Revendications

1. Procédé de fabrication d'acide 2-méthylbutyrique ayant une teneur réduite en acide 3-méthylbutyrique à partir des courants secondaires formés lors de la fabrication d'acides pentanoïques, **caractérisé en ce que**
a) un mélange contenant des butènes linéaires est mis en réaction en présence de composés de métaux de transition du groupe VIII du tableau périodique des éléments avec du monoxyde de carbone et de l'hydrogène à température élevée et pression élevée pour former un mélange de pentanals ;
b) le mélange obtenu selon l'étape a) est séparé en un courant de matières enrichi en 2-méthylbutanal et 3-méthylbutanal et en un courant de matières enrichi en n-pentanal ;
c) le courant de matières enrichi en 2-méthylbutanal et 3-méthylbutanal selon l'étape b) est mis en réaction avec du formaldéhyde ;
d) le produit de réaction obtenu selon l'étape c) est traité avec un oxydant ; et
e) à partir du produit de réaction obtenu selon l'étape d), de l'acide 2-méthylbutyrique ayant une teneur résiduelle en acide 3-méthylbutyrique de moins de 0,2 % en poids, par rapport à la fraction organique, est éliminé par distillation ;
à condition- que la phase organique soit séparée après la réaction avec le formaldéhyde selon l'étape c) et hydrogénée sélectivement en présence d'un catalyseur d'hydrogénation avec de l'hydrogène à température élevée et pression élevée, et la sortie d'hydrogénation obtenue est traitée après la séparation du catalyseur d'hydrogénation avec un oxydant selon l'étape d).

2. Procédé selon la revendication 1, **caractérisé en ce que** la réaction avec le formaldéhyde selon l'étape c) a lieu en présence d'une amine secondaire et d'un acide mono-, di- ou polycarboxylique.

3. Procédé selon la revendication 1, **caractérisé en ce que** la sortie d'hydrogénation obtenue est soumise à une distillation fractionnée après la séparation du catalyseur d'hydrogénation, et les fractions séparées sont traitées avec un oxydant selon l'étape d).

4. Procédé selon une ou plusieurs des revendications 1 à 3, **caractérisé en ce qu'**une alkylamine de formule R¹-NH-R² est utilisée en tant qu'amine secondaire, R¹ et R² étant identiques ou différents, et représentant des radicaux alkyle de 1 à 12, de préférence 3 à 5 atomes de carbone.

5. Procédé selon une ou plusieurs des revendications 1 à 4, **caractérisé en ce qu'**un acide monocarboxylique de 1 à 10 atomes de carbone ou un acide di- ou polycarboxylique de 2 à 10 atomes de carbone est utilisé.

6. Procédé selon une ou plusieurs des revendications 1 à 5, **caractérisé en ce que** l'oxydation à l'étape d) a lieu en présence de carboxylates de métaux alcalins ou alcalino-terreux.

7. Procédé selon la revendication 6, **caractérisé en ce que** des carboxylates de lithium, de potassium, de sodium, de calcium ou de baryum sont utilisés en tant que carboxylates de métaux alcalins ou alcalino-terreux.

8. Procédé selon une ou plusieurs des revendications 1 à 7, **caractérisé en ce que** l'oxydation a lieu dans la phase liquide à l'étape d).

9. Procédé selon une ou plusieurs des revendications 1 à 8, **caractérisé en ce que** de l'oxygène ou des gaz contenant de l'oxygène sont utilisés en tant qu'oxydant à l'étape d).

10. Procédé selon une ou plusieurs des revendications 1 à 9, **caractérisé en ce que** la réaction avec du formaldéhyde est réalisée en continu dans un tube d'écoulement à l'étape c).
